# EUROPEAN PATENT APPLICATION

(11) **EP 3 996 102 A1**
(43) Date of publication of application: **11.05.2022**
(21) Application number: 20206290.7
(22) Date of filing: 06.11.2020
(51) Int. Cl.: G16H 30/00, G16H 50/20, G16H 50/70

(54) **METHOD FOR DETECTION OF NEUROLOGICAL ABNORMALITIES**

(71) Applicant: Windisch, Paul Yannick, 8610 Uster (CH)
(72) Inventor: Windisch, Paul Yannick, 8610 Uster (CH)
(74) Representative: P&TS SA (AG, Ltd.)

(57) **Abstract**

The invention discloses a computer-implemented method for identifying pathological abnormalities comprising training a first artificial neural network (1) to classify automatically MR images or series of MR images according to their perspective and/or acquisition sequence, and classifying clinical MR data from patients by automatically determining perspectives and/or acquisition sequences of the clinical MR data with the first artificial neural network (1), then classifying the clinical MR data for abnormal conditions using the perspectives and/or acquisition sequences determined automatically.

## Description

### Technical domain

The present invention concerns an automated classification method for medical magnetic resonance images, which is suited for automated detection of neurological pathologies.

### Related art

Research on the application of deep learning on magnetic resonance (MR) images to detect, classify and contour brain tumors has been conducted for several years and achieved impressive initial results in many cases. However, the amount of research that has been translated into clinical application is limited. One of the reasons for this is the lack of methods with the capacity to deliver similarly reliable processing results for MR image data obtained from a variety of different radiology laboratories, using different scanners, and the differences in data acquired for different clinical indications.

Automated diagnostic methods for MR images are usually developed to interpret specific types of data sets. However, an MRI study often consists of different series which can vary significantly between different radiology practices as well as between different indications. When faced with a large palette of heterogenic MR data sets, automated methods may not always be equipped to reliably evaluate the different types of MR data.

In the prior art a series of different approaches has been described to employ deep-learning algorithms for image segmentation and classification, in order to arrive at a diagnostic output regarding the pathology status of a scanned body portion.

Document US2019/0320934 discloses an automated sequence prediction method for medical imaging including a self-assessment mechanism. The method describes the acquisition of an initial scout sequence based on a first imaging protocol. Based on the validation of the first MR data a processor orders a second imaging protocol. This step can be repeated to fine tune the acquisition protocol. Optionally, a deep-learning method can be employed to select appropriate setting and procedures. While these method is suited to optimise an acquisition protocol of a particular scanner, it does not teach how to reliably classify and subsequently analyse MR images which were obtained from different scanners or laboratories.

Document US2020027561 discloses a method for neurological tumour identification based on a multilayer convolutional network, which is configured to segment image data sets of full neurologic scans into (3D) resolution voxels. The method employs a supervised learning and validations platform for classification of tissue within classification voxels having a ground truth, whereby the ground truth refers to the pathology status of the tissue. The method does however not ensure that MR image sets from a variety of sources can be diagnosed with the same reliability despite their inherent differences due to varying acquisition protocols.

Document US10223610 discloses a method for detecting and classifying multiple findings in images based on a number of convolutional and pooling layers of a neuronal network producing a plurality of feature maps, which are then used to define regions of interest pools, which are subsequently combined by concatenation. This ROI is then processed by a classification network to produce an output result. The method aims to classify an image feature and to localise said feature in an image.

Document WO2006114003 describes a method for automated detection and segmentation of tumours based on aligning input MR images comprising a plurality of pixels with template images. Segmentation of objects represented in the input images is subsequently performed according to the classification of each pixel or group of pixels. The classification is based on template images.

Document CN109035263 discloses a convolutional network-based tumour segmentation of multi-modal MR images employing a pre-processing step to obtain an original image set and, subsequently, modular segmentation of the image set. Module 1 comprises a parallel deep deconvoluting neural network based on a 3D convolution neural network, a residual unit and a transposed convolution, while module 2 includes adding a hopping structure. Pre-processing of the sample comprises correction by N4ITK and adjusting the contrasts of the images.

Documents CN107016681 also disclose convolutional network-based tumour segmentation of MR images employing a pre-processed sample to detect a tumour region in original image. Segmentation is subsequently performed in a coarse segmentation step and in a subsequent fine segmentation step performed by a trained convolutional network. The fine segmentation is performed on the internal structure of the tumour region.

Both, document CN109035263 and document CN107016681 rely on sample pre-processing in order to perform image segmentation.

Despite the multitude of approaches to automated MR image analysis based on neural networks and deep learning methods, the basic problem of how an automated method can efficiently perform a reliable diagnosis of heterogenous real world MR data sets is still not sufficiently addressed. In other words, there is a need for an automated diagnostic MR image processing method, which can efficiently deal with the heterogenicity of MR input images and deliver reliable diagnoses despite said heterogenicity.

### Short disclosure of the invention

An aim of the present invention is the provision of a computer-implemented method for automated MR image analysis, which overcomes the shortcomings and limitations of the state of the art and achieves the above stated objective.

According to the invention, these aims are attained by the object of the attached claims.

In a preferred variant, the object of the invention is achieved by a computer-implemented method for identifying pathological abnormalities comprising training a first neural network to classify MR images or series of MR images according to their perspective and/or acquisition sequence, and further classify clinical MR data from patients by automatically determining perspective and/or acquisition sequence of the clinical MR data with a first neural network, then examining the clinical MR data for abnormal conditions using the perspective and/or acquisition sequence determined automatically.

The training of the first neural network may involve a learning task on training data including MR images or series thereof as input objects, and perspectives and/or acquisition sequences as desired results. The neural network may be a deep network, preferably a convolutional one, and may be a network pretrained for generic image classification and retrained for determining perspectives and/or acquisition sequences in the above manner.

The determination of the perspective and/or acquisition sequence of the clinical MR data can be done on a predetermined number, for example between 1 and 5, preferably between 1 and 3, of MR slices, ideally on a single MR slice.

The analysis for abnormal conditions may be delegated to a specialized neural network, chosen among a plurality of neural networks based on the perspective and/or acquisition sequence determined by the first neural network.

Each of the specialized neural networks can be trained previously with further training data comprising MR images or sets of MR images having a given perspective and/or acquisition sequence as input objects, and pathological conditions as desired output values. In this manner, the method of the invention can choose, based on the automatically determined perspective and/or acquisition sequence, a neural network especially trained for classifying clinical MR data having that perspective and/or acquisition sequence.

The useful classes that the first neural network could have as possible output values are: sagittal, axial, and coronal, for the perspective; T1-weighted without contrast enhancement, T1-weighted with contrast enhancement, T2-weighted, T2-weighted Fluid attenuated inversion recovery (FLAIR) and diffusion-weighted for the acquisition sequence, as these are the most employed sequences in neuro-oncology. Other acquisition sequences may be added or substituted, according to the use case.

In the context of this disclosure, the term "deep learning" refers to machine learning methods aimed at learning feature hierarchies, whereby features of higher level of hierarchy are formed by compositions of features of lower level of hierarchy. "Deep learning" includes machine learning methods based artificial neural networks with multiple layers between an input layer and an output layer, also denoted as "deep neural network".

Due to the use of neural networks for the definition of different classes of acquisition sequences and/or perspective of clinical MR data, the inventive method is suitable for correct interpretation and segmentation of heterogeneous input data.

The term "image segmentation" as used herein means a process of processing an MR image or series of images wherein certain qualities of the images are not only detected as present or absent in the image as a whole, but also in individual pixel or local structures in the image.

In the context of automatic diagnostic tools of MRI images, segmentation methods may provide an outline of the structures in the image, in particular abnormal structures. Image structures are used for classification of the image. An image can be classified according to predefined conditions, for example abnormal conditions such as neoplasms or lesions.

The term "heterogeneous" as used herein means that the MR image data or data sets stem from different radiology laboratories using a variety of radiological practices and equipment, in particular scanners, and/or which are scans of different patients having different indications. A specific combination of these variations is hereinafter referred to as specific "circumstances". These heterogenicity corresponds to real world data, which may be received for automated analysis. An automated method for image analysis, which is trained to adequately segment and classify these real world data with respect to their perspective and their acquisition sequences provides a sound basis to further process the images according to their class. Misinterpretations in analysis of the MR image data, respectively data sets, which are due to an inaccurate interpretation of the perspective or the acquisition sequence are therefore avoided. This is a distinctive advantage over existing methods when handling a large variety of MR images which were generated under different circumstances.

Preferred body parts to be scanned are specific parts of the nervous system, such as the brain or the spinal cord. The neurological abnormalities refer preferably to neoplastic transformations. However, the invention is not limited to the mentioned preferred examples, but is suitable to perform reliable analyses of a variety of scans of different body portions as well as to detect a variety of different abnormalities, such as, by way of example, abnormalities with respect to myelinization of nerve fibres, degeneration of nerve fibres, vascularisation of the scanned body part, and others.

Since the classifiers delimitate different classes having a certain range of variability in radiographic features, the classifiers may be trained to recognise whether particular radiographic features of an input sample are either less distinct or overemphasized in relation to an average sample. By way of example, such radiographic features include, without limitation, absolute voxel or pixel intensities within the scanned region as well as relative voxel or pixel intensities of different tissues in comparison to each other resulting in different grey levels etc. The differences between a real world sample and a calculated average of each category can then be accounted for, respectively compensated, in the further processing steps, for example with the use of a generative adversarial network (GAN), a type of neural network that is able to create new data while keeping certain properties of one or several training sets. This again improves the reliability of the diagnostic result.

The automated method presented herein is capable of correctly characterising MR images, which are not necessarily consistent with a preferred type of MR image, i.e. different images, which differ from the type of images obtained under specific preferred circumstances. Since the different classes allow for deviations from a specific preferred type of image, the method does not necessarily require an entire series of MR images in order to be able to identify the perspective and/or acquisition sequence displayed in the scan of a body part. Instead, one or a limited number of MR images are enough to proceed with further classification and segmentation steps.

With respect to what is known in the art, the invention therefore provides the advantage that is suited to perform a reliable analysis of the MR scan based in either only one image or on a limited number of serial MR images. This limited number of images is typically less than 5 images, preferably less than 3 images per series.

The trained classifiers define the delimitation of the pre-selected classes, which are perspectives classes for the perspective classifier and acquisition sequence classes for the sequence classifier. The classifier is capable to identify if a sample data sets corresponds to an average representative data set of a particular category, or if such data set displays features which correspond more to an extreme within this category. The classifier is capable of relaying said information to allow for an adaptation of the further image segmentation steps according to this information.

Preferably the perspective classifier distinguishes between at least two, preferably three, different perspective classes, preferably an axial, a coronal and/or a sagittal category.

Preferably the sequence classifier distinguishes between at least two different classes, such as for example a T1-weighted, a T1-weighted with contrast, a T2-weighted, a T2-weighted fluid attenuated inversion recovery (FLAIR) and/or a diffusion-weighted category.

One of the main advantages of this method, i.e. the reliable interpretation of heterogeneous real world data, resides therefore primarily on the provision of separate neural networks, the classifiers, which are trained to distinguish particular perspectives and acquisition sequences. The classifiers only pass the relevant MR data or data sets to a fitting subsequent neural network which was trained to further segment the data, in order to arrive at an output result. Such output result is preferably the identification of the pathological status, preferably of a neurological abnormality. A fitting subsequent neural network is a network which is trained to process samples having a defined combination of perspective and acquisition sequence.

Different automated processing steps and/or methods for further analysis of the one or more MR images are possible. Preferably, such method is based on one or more specialized neural network, which are trained to perform segmentation on the input MR images of a particular combination of associated sequence and perspective category and which are configured to detect abnormalities in the input MR images and to generate output data.

In a preferred subsequent step, said output data are then merged by means of an ensemble method in order to generate data relating to each region of feature of an MR image or a series of MR images. The ensemble method can range from a simple approach such as a majority vote of the specialized networks on which abnormality is present to more complex approaches such as training a separate neural network to take predictions from the specialized networks as input and identify the most likely abnormality as its output.

The method is particularly suited for clinical diagnosis of tumours of the central nervous system, for example brain tumours or tumours of the spinal cord.

In addition, the method is preferably suited for detecting tumorous tissue, distinguishing tumorous tissue from non-tumorous tissue, characterising the tumour based on size, shape, location, homogeneity, compression of adjacent structures, invasion of adjacent structures and/or contrast agent uptake, and, optionally, associating the tumour with defined tumour classes.

Diagnostic results are preferably associated with specific classes, which may, by way of example, correspond to malignant tumors (in case of imaging of the central nervous system: glioma, high-grade meningioma, metastases), benign tumors (vestibular schwannomas, low-grade meningioma), arteriovenous malformations, aneurysms etc.

### Short description of the drawings

Exemplar embodiments of the invention are disclosed in the description and illustrated by the drawings in which:
**Figure 1** illustrates schematically a preferred embodiment of a sample prediction workflow for tumour segmentation.
**Figure 2** shows correctly classified example images in the respective axial, coronal and sagittal perspective classes.
**Figures 3a,b,c, and d** show confusion matrices for the perspective classifier and for the sequence classifier, wherein

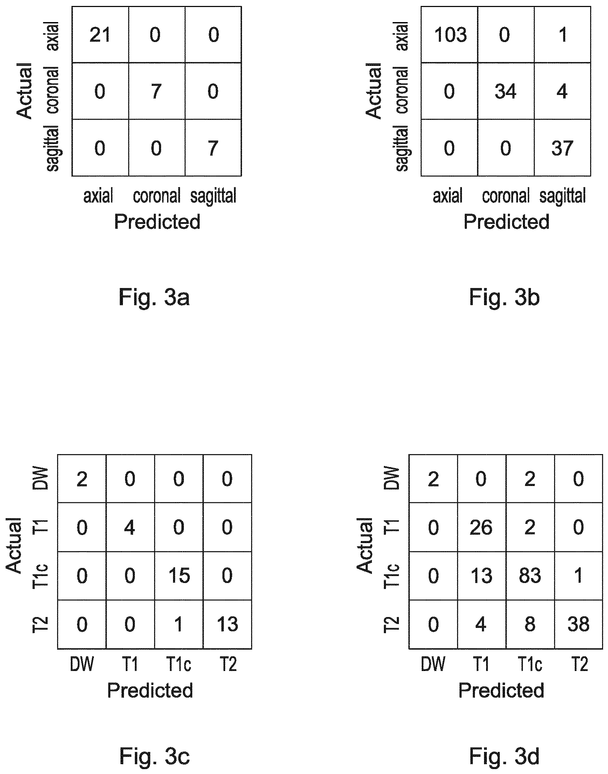

   **Figure 3a** shows a confusion matrix of the perspective classifier results for a previously unseen glioblastoma data set,
   **Figure 3b** shows a confusion matrix of the perspective classifier results for a previously unseen brain metastases data set,
   **Figure 3c** shows a confusion matrix of the sequence classifier results for a previously unseen glioblastoma data set, and
   **Figure 3d** shows a confusion matrix of the sequence classifier results for a previously unseen brain metastases data set.

### Examples of embodiments of the present invention

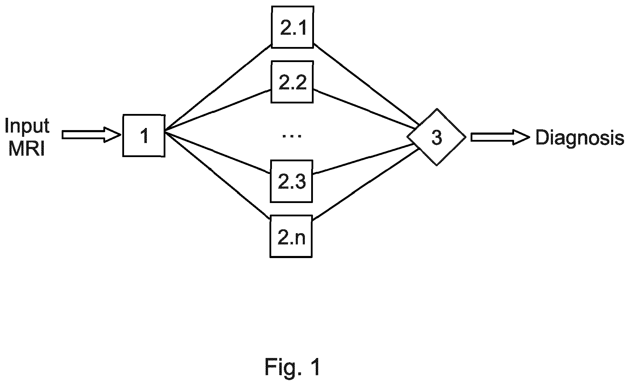

With reference to Figure 1, a preferred embodiment of the automated MR image classification method is schematically presented, wherein the perspective and the sequence classifiers are together depicted as a perspective/sequence detection network 1.

In order to be able to classify MR images obtained from a plethora of different sources and radiology laboratories the sequence classifier and the perspective classifier are preferably trained using heterogenous MR image data.

In a preferred embodiment the perspective/sequence detection network 1 is a convolutional neural network, preferably a pretrained convolutional neural network.

Training of the perspective/sequence detection network 1 may comprise the following steps:
1. collecting a plurality of heterogeneous data sets of MR images from scans of the body portion of interest,
2. defining a plurality of different MR image classes based on
   i. the perspective of the body portion visualised by the MR image, the perspective classes, and on
   ii. the acquisition sequences of the MR scan, the sequence classes,
3. training the perspective classifier and the sequence classifier to perform segmentations on the basis of the acquisition sequences and of perspectives of said heterogenous data sets in order to delimitate the scope of each class.

Once training is completed, the perspective/sequence detection network 1 is used to classify MR images, regardless from which laboratory the data sets originate or which type of MR scanner was used to obtain the images.

It is possible, that MR images obtained for training or analysis purposes are already pre-labelled with a specific condition, for example a tumour category. Preferably, the perspective/sequence detection network 1 maintains this label prior to performing classification according to acquisition sequence and perspective.

Classification of MR images performed by the perspective/sequence detection network 1 may comprise the following steps:
1. obtaining one or a series of MR images of a patient's body part of interest for analysis,
2. employing the trained sequence classifier to detect the acquisition sequence of each MR image or of the series of MR images and to assign each MR image or series of MR images to a fitting sequence class,
3. employing the trained perspective classifier to detect the perspective of each MR image or of the series of MR images and to assign each MR image or series of MR images to a fitting perspective category.

Further analysis of the classified MR images are then preferably performed by a number of distinct specialized neural networks 2.1, 2.2, 2.3, 2.n. Preferably each specialized neural network 2.1, 2.2, 2.3, 2.n is trained using images from one particular combination of perspective and acquisition sequence, for example images classified as T1-weighted (acquisition sequence) and axial slices (perspective sequence). Each specialized neural network 2.1, 2.2, 2.3, 2.n is preferably trained with images of a plurality of different conditions, e.g. a specific types of tumours.

As a result of this preferred training, each specialized trained network 2.1, 2.2, 2.3, 2.n is optimised for the processing and/or analysis of MR images with a particular combination of acquisition sequence and perspective, in order to reliable predict abnormal conditions, e.g. a particular type of tumour.

Following their initial classification by the the perspective/sequence detection network 1, MR image data may then be received by the appropriate specialized network 2.1, 2.2, 2.3, 2.n, which performs further image processing, preferably image segmentation, on one or a series of the sequence- and perspective-classified MR images.

The further image analysis steps performed by the specialized networks preferably result in a preliminary prediction. Since each specialized network 2.1, 2.2, 2.3, 2.n processes MR images having one particular combination of acquisition sequence and perspective view only, each prediction is specific for the patient samples having this particular perspective view and acquisition sequences.

In order to arrive at a reliable diagnosis, radiologists usually scan the patient body area of interest from a number of different perspectives and using different acquisition sequences. As a consequence, for each analysis a series of different MR images, i.e. images taken from varying perspectives and having been acquired using different sequences, is usually obtained. Therefore, a number of different specialized networks usually are employed to process each different series. Independently from one another, each specialized network 2.1, 2.2, 2.3, 2n then arrives at a preliminary predictive result regarding the condition of the analysed sample.

If all networks were perfectly trained, all networks would arrive at the same conclusion. In practice, this is rarely the case, which is why the described method preferably includes a further step as part of which the results which were predicted by the different specialized networks are evaluated and merged to arrive at a final output. This step is particularly important in cases where specialized networks do not agree on the conclusion regarding the predicted condition.

In the preferred embodiment depicted in Figure 1, a further evaluation step 3, preferably based on an ensemble method is used to arrive at one final prediction.

As mentioned above, an MR study of a patient comprises different series (axial T1 with contrast, sagittal T2 etc.) of images. Tumors look differently in the different series and each series provides information to the radiologist. A radiologist considers the different imaging series in order to arrive at a diagnosis regarding the medical condition of the patient.

Similarly, the ensemble method 3 takes all predictions received from the specialized networks 2.1, 2.2, 2.3, 2n into account in order to determine the condition visualised in the MR scans. If all specialized networks 2.1, 2.2, 2.3, 2n agree on the predictive result, the analysis is straightforward.

However, if the specialized networks 2.1, 2.2, 2.3, 2n do not agree, the evaluation step 3 should provide for a reliable method to calculate and/or decide on the final prediction based on the input results received from the specialized networks 2.1, 2.2, 2.3, 2n. This step may be performed by using the ensemble method. By way of example, the ensemble method 3 may be based on a simple majority vote. The ensemble method 3 may also be based on a separate neural network, which is trained to take predictions from the specialized networks 2.1, 2.2, 2.3, 2n as input and identify the most likely prediction as its output.

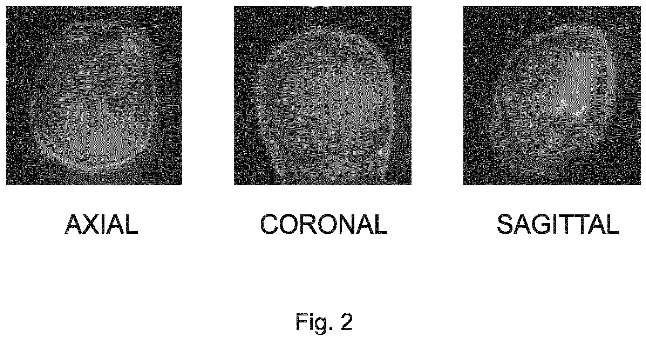

In Figure 2 a number of MR scans classified according to their respective perspective views are shown. Preferably, the perspective classifier is trained to allocate samples to the habitual categories of axial, coronal and sagittal perspective views.

Figures 3a,b,c,and d shows results of inital experiments performed to test the proposed classification method. The experiments were conducted to train perspective/sequence detection network 1 to classify perspectives and acquisition sequences of brain MR images based on a single slice.

Confusion matrices for the perspective classifier and acquisition sequence classifier on previously unseen test data patient scan datasets are shown.

Figures 3a and 3b shows the confusion matrices for the perspective classifier, whereby in Figure 3a the classifier was presented with previously unseen glioblastoma datasets (35 total images) and in Figure 3b the classifier was presented with previously unseen brain metastases dataset (179 total images). Figures 3c and 3d show the matrices for the trained sequence classifier for the same glioblastoma (Figure 3c) and brain metastasis (Figure 3b) datasets respectively. The references used in Figures 3c and 3d have the following meaning: DW = diffusion-weighted, T1 = T1-weighted, T1c = T1-weighted with contrast enhancement, T2 = T2-weighted.

For this experiment a pretrained convolutional neural network (ResNet50) was used as part of a transfer learning approach and retrained on MRI slices that were labelled with perspective (axial, coronal, sagittal) and acquisition sequence (T1-weighted without contrast enhancement, T1-weighted with contrast enhancement, T2-weighted and diffusion-weighted).

A total of 409 MRI slices from 63 different patients were created from the The Cancer Genome Atlas (TCGA) glioblastoma (GBM) dataset provided as part of The Cancer Imaging Archive (TCIA). All 409 slices were used for the development of the perspective classifier neural network. For the acquisition sequence classifier neural network, only slices belonging to the four aforementioned classes were included while other acquisition sequences were discarded, resulting in 338 slices.

Prior to training, 10% of slices were removed to later serve as unseen test data. Of the remaining slices, 80% were used for training and 20% for validation. Resampling of the slices was performed to balance the different category sizes. Data augmentation was performed by applying rotation, shift, zoom, horizontal flip and different brightness to the images.

Both the perspective classifier as well as the sequence classifier were trained with 160 training slices and 32 validation slices per category after resampling. Training was performed for 100 epochs using a batch size of 50 and an Adam optimizer with learning rate 10⁻⁴. Following training and validation, testing on the previously unseen 10% of slices was conducted.

In a second step, external validation was conducted. This validation included testing the network on 179 slices created from 19 MRIs of patients with brain metastases acquired on various scanners and provided by the European Cyberknife Center in Munich. To resemble real world data, slices were exported from all available series that belonged to the classes the model had been trained on which lead to an overrepresentation of axial and T1-weighted slices in the dataset (perspective: 104 axial, 38 coronal, 37 sagittal; sequence: 28 T1-weighted, 97 T1-weighted with contrast enhancement, 50 T2-weighted, 4 diffusion-weighted).

Ultimately, Grad-CAM was employed to identify regions that the predictions of the networks were based on.

The testing on the previously unseen images from the same dataset resulted in a categorical accuracy of 1.00 for the perspective classifier and 0.97 for the sequence classifier

The external validation of the model resulted in a categorical accuracy of 0.97 for the perspective- and 0.83 for the sequence classifier.

While T1-weighted images were almost never confused for T2- or diffusion-weighted images, T2-weighted images were several times falsely classified as T1-weighted, especially T1-weighted with contrast-enhancement.

The implementation of Grad-CAM showed a tendency of the perspective classifier to focus on the brain itself while the acquisition sequence classifier focussed on the adjacent structures. However, no consistent pattern could be found that applies to the whole set of slices.

The initial proof-of concept study demonstrates, that the disclosed methods provides for a reliable prediction of the medical condition based on analysis of single MR scans. The ability to confidently make predictions on single MR scans as opposed to using a whole series of scans has the advantage regarding the application to real world data that only one image from a series of an MRI needs to be sampled in order to determine perspective and acquisition sequence which reduces the need for computational power.

It is to be expected, that by collecting more MR image data to train the perspective/sequence detection network 1, te method will be further enhanced and the automatic detection of perspective and acquisition sequence will further improve on reliability.

### References in the figures

- 1: perspective/sequence detection network
- 2: specialised neuronal networks (2.1, 2.2, 2.3 and 2.n)
- 3: evaluation step
- DW: diffusion-weighted
- T1: T1-weighted
- T1c: T1-weighted with contrast enhancement
- T2: T2-weighted

## Claims

1. A computer-implemented method for identifying pathological abnormalities comprising training a first artificial neural network (1) to classify automatically MR images or series of MR images according to their perspective and/or acquisition sequence, and classifying clinical MR data from patients by automatically determining perspectives and/or acquisition sequences of the clinical MR data with the first artificial neural network (1), then classifying the clinical MR data for abnormal conditions using the perspectives and/or acquisition sequences determined automatically.

2. The computer-implemented method of the preceding claim, wherein the training of the first artificial neural network (1) includes a learning task on training data including MR images or series thereof as input objects, and perspectives and/or acquisition sequences as desired results.

3. The computer-implemented method of any one of the preceding claims, wherein the training data are obtained from different radiology laboratories using different radiological practices and/or equipment and/or which are scans of different patients having different indications.

4. The computer-implemented method of any one of the preceding claims, wherein the first artificial neural network (1) is a deep neural network or a convolutional neural network.

5. The computer-implemented method of any one of the preceding claims, wherein the first artificial neural network (1) is pretrained for generic image classification and retrained to classify MR images or series of MR images according to their perspective and/or acquisition sequence.

6. The computer-implemented method of any one of the preceding claims, wherein the training of the first artificial neural network (1) is supervised.

7. The computer-implemented method of any one of the preceding claims, wherein the step of classifying MR data for abnormal conditions includes sending the clinical MR data to the input of a specialized neural network (2.1, 2.2, 2.3, 2.n) trained to classify abnormal conditions in MR data having the same perspective and/or acquisition sequence as the perspective and/or acquisition sequence determined automatically.

8. The computer-implemented method of the preceding claim, wherein the one or more specialized neural networks (2.1, 2.2, 2.3, 2.n) are prior trained with further training data comprising MR images or sets of MR images having a determined perspective and/or acquisition sequence as input objects, and pathological abnormalities as desired output values.

9. The computer-implemented method of any one of the preceding claims, wherein the first artificial neural network (1) determines the perspective and/or the acquisition sequence of clinical MR data based on a predetermined number of 1 to 5, preferably 1 to3, MR slices, ideally based on a single MR slice.

10. The computer-implemented method of any one of the preceding claims, wherein the output classes of the first artificial neural network (1) include for the perspective: sagittal, axial, and coronal and/or for the acquisition sequence: T1-weighted with contrast enhancement, T1-weighted without contrast enhancement, T2-weighted, T2-weighted Fluid attenuated inversion recovery (FLAIR) and diffusion-weighted.

11. The computer-implemented method of any one of the preceding claims, wherein the abnormal conditions are determined in neurological tissue, preferably in the central nervous system.

12. The computer-implemented method of any one of the preceding claims, wherein the abnormal condition includes brain neoplasms and/or brain lesions.

13. The computer-implemented method of any one of the claims 7 to 11, wherein the clinical MR data contain MR images or series of MR images acquired under a plurality of perspectives and/or acquisition sequences that are sent to the input of a plurality of specialized neural networks (2.1, 2.2, 2.3, 2.n) according to their respective automatically determined perspectives and/or acquisition sequences.

14. The computer-implemented method of claim 13, wherein the method further comprises an ensembling step (3) combining the output of the specialized neural networks (2.1, 2.2, 2.3, 2.n) to provide an ensemble classification for abnormal conditions.

15. The computer-implemented method of any one of the preceding claims, further comprising segmentation and outlining of abnormal structures in the MR images or series of MR images.
